(19) Europäisches Patentamt
European Patent Office
Office européen des brevets

(11) **EP 4 487 809 A1**

(12) **EUROPEAN PATENT APPLICATION**
published in accordance with Art. 153(4) EPC

(43) Date of publication:
08.01.2025 Bulletin 2025/02

(21) Application number: 23763715.2

(22) Date of filing: 02.03.2023

(51) International Patent Classification (IPC):
A61B 34/30 (2016.01)      A61B 17/22 (2006.01)
B25J 11/00 (2006.01)      B25J 15/00 (2006.01)
B25J 15/02 (2006.01)

(52) Cooperative Patent Classification (CPC):
A61B 17/22; A61B 34/30; B25J 11/00; B25J 15/00;
B25J 15/02

(86) International application number:
PCT/KR2023/002887

(87) International publication number:
WO 2023/167517 (07.09.2023 Gazette 2023/36)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 04.03.2022 KR 20220027983

(71) Applicant: IUCF-HYU (INDUSTRY-UNIVERSITY
COOPERATION
FOUNDATION HANYANG UNIVERSITY)
Seoul 04763 (KR)

(72) Inventors:
• JANG, Gun Hee
  Seoul 06326 (KR)
• SA, Jun Chi
  Seoul 02444 (KR)
• PARK, Ji Min
  Seoul 05208 (KR)
• KWON, Jun Hyoung
  Seoul 04762 (KR)

(74) Representative: Prüfer & Partner mbB
Patentanwälte · Rechtsanwälte
Sohnckestraße 12
81479 München (DE)

(54) **MAGNETIC ROBOT**

(57) The present invention relates to a magnetic robot. The magnetic robot according to one embodiment of the present invention comprises: a body; and a work member which is connected to the body so as to be positioned in an anterior region of the body, and which is provided to be rotatable around a longitudinal center as an axis, wherein the body comprises: a housing part provided to have a preset length in the forward-backward direction so as to form an inner space; and a connection part positioned at a front end portion of the housing part so as to be provided to protrude toward the longitudinal central axis of the housing part, and thus has a connection hole formed at the inner center thereof, and the work member comprises: a body part which is provided to have a preset length in the forward-backward direction, and which has at least one region provided as a magnetic body; and a fastening part provided to extend backward from the rear end of the body part so as to be inserted into the connection hole.

FIG. 1

**Description**

[Technical Field]

The present invention relates to a magnetic robot, and more particularly, to a magnetic robot capable of moving in a blood vessel while effectively steering a front region of the magnetic robot, and effectively fragmenting and removing a thrombus through a drilling work.

[Background Art]

Magnetic catheters are driven with a magnetic torque and a magnetic force by an external magnetic field generated by a magnetic driving system, and the magnetic catheters may be precisely controlled remotely, so that the magnetic catheters are being applied to many regions, and research and development on the magnetic catheters are being conducted.

A representative example is a magnetic catheter applied to a treatment of cardiac arrhythmia, and a blood vessel treatment magnetic catheter for a blood vessel treatment is being developed. In a case of the blood vessel treatment magnetic catheter, it is very important to perform a tunneling treatment function to unclog a solidly clogged blood vessel. Existing magnetic catheters perform a tunneling treatment by rotating a drill tip and allowing a person to push the magnetic catheter outside a body by using a hand or a linear slide mechanism.

Such magnetic catheters have a limitation in that when a lesion is positioned after a complex blood vessel, a force may not reach an end of the catheter so that the tunneling treatment may not be effectively performed.

[Disclosure]

[Technical Problem]

An object of the present invention is to provide a magnetic robot capable of moving in a blood vessel while effectively steering a front region of the magnetic robot, and effectively fragmenting and removing a thrombus through a drilling work.

In addition, an object of the present invention is to provide a magnetic robot capable of adjusting a posture such that a longitudinal direction of a work member is inclined with respect to a longitudinal direction of a body.

In addition, an object of the present invention is to provide a magnetic robot capable of adjusting a position of a work member in a forward-rearward direction with respect to a body.

[Technical Solution]

According to one aspect of the present invention, there is provided a magnetic robot including: a body; and a work member connected to the body so as to be positioned in an anterior region of the body, and provided to be rotatable about a longitudinal center of the work member, wherein the body includes: a housing part having a preset length in a forward-rearward direction so as to form an inner space; and a connection part positioned at a front end portion of the housing part so as to protrude toward a longitudinal central axis of the housing part, and having a connection hole formed at an inner center of the connection part, and the work member includes: a body part having a preset length in the forward-rearward direction, and having at least one region provided with a magnetic body; and a fastening part extending rearward from a rear end of the body part so as to be inserted into the connection hole.

In addition, the work member may further include: a separation prevention part coupled to a rear end of the fastening part so as to be positioned in the inner space of the housing part, and having at least a partial region in which a section of the partial region, which is perpendicular to a longitudinal direction of the work member, is larger than an area of the connection hole.

In addition, a section of the connection hole, which is perpendicular to a longitudinal direction of the connection hole, may have a circular shape, a section of the fastening part, which is perpendicular to a longitudinal direction of the fastening part, may have a circular shape, and a diameter of the fastening part may be smaller than a diameter of a central region of the connection hole by a preset length.

In addition, a diameter of the connection hole may be larger in a front end portion of the connection part than in a longitudinal central region of the connection part.

In addition, the front end portion of the connection part may have a structure that widens outward from a rear side to a front side.

In addition, a diameter of the connection hole may be larger in a rear end portion of the connection part than in a longitudinal central region of the connection part.

In addition, the rear end portion of the connection part may have a structure that widens outward from a front side to a rear side.

In addition, at least one communication hole may be formed at an outer circumference of the housing part.

In addition, the communication hole may be inclined forward at a preset angle from the inner space to an outside.

[Advantageous Effects]

According to one embodiment of the present invention, a magnetic robot capable of moving in a blood vessel while effectively steering a front region of the magnetic robot, and effectively fragmenting and removing a thrombus through a drilling work can be provided.

In addition, according to one embodiment of the present invention, a magnetic robot capable of adjusting a posture such that a longitudinal direction of a work member is inclined with respect to a longitudinal direction of a body can be provided.

In addition, according to one embodiment of the present invention, a magnetic robot capable of adjusting a position of a work member in a forward-rearward direction with respect to a body can be provided.

[Description of Drawings]

FIG. 1 is a view showing a magnetic robot according to one embodiment of the present invention.

FIG. 2 is a view showing a section of one portion of the magnetic robot according to one embodiment of the present invention.

FIGS. 3 and 4 are views showing states where a direction in which a work member faces is adjusted.

FIGS. 5 and 6 are views showing states where a position of the work member is adjusted in a forward-rearward direction with respect to a body.

FIG. 7 is a view showing a method for inserting a magnetic robot into a blood vessel to perform a work.

FIG. 8 is a view showing a magnetic robot according to a second embodiment of the present invention.

FIG. 9 is a view showing a magnetic robot according to a third embodiment of the present invention.

FIG. 10 is a view showing a magnetic robot according to a third embodiment of the present invention.

[Best Mode]

According to one embodiment of the present invention, a magnetic robot includes: a body; and a work member connected to the body so as to be positioned in an anterior region of the body, and provided to be rotatable about a longitudinal center of the work member, wherein the body includes: a housing part having a preset length in a forward-rearward direction so as to form an inner space; and a connection part positioned at a front end portion of the housing part so as to protrude toward a longitudinal central axis of the housing part, and having a connection hole formed at an inner center of the connection part, and the work member includes: a body part having a preset length in the forward-rearward direction, and having at least one region provided with a magnetic body; and a fastening part extending rearward from a rear end of the body part so as to be inserted into the connection hole.

[Mode for Invention]

Hereinafter, exemplary embodiments of the present invention will be described in detail with reference to the accompanying drawings. However, the technical idea of the present invention is not limited to the embodiments described herein, but may be embodied in different forms. The embodiments introduced herein are provided to sufficiently deliver the idea of the present invention to those skilled in the art so that the disclosed contents may become thorough and complete.

When it is mentioned in the present disclosure that one element is on another element, it means that one element may be directly formed on another element, or a third element may be interposed between one element and another element. Further, in the drawings, thicknesses of films and regions are exaggerated for effective description of the technical contents.

In addition, although the terms such as first, second, and third have been used to describe various elements in various embodiments of the present disclosure, the elements are not limited by the terms. The terms are used only to distinguish one element from another element. Therefore, an element mentioned as a first element in one embodiment may be mentioned as a second element in another embodiment. The embodiments described and illustrated herein include their complementary embodiments, respectively. Further, the term "and/or" used in the present disclosure is used to include at least one of the elements enumerated before and after the term.

As used herein, an expression in a singular form includes a meaning of a plural form unless the context clearly indicates otherwise. Further, the terms such as "including" and "having" are intended to designate the presence of features, numbers, steps, elements, or combinations thereof described herein, and shall not be construed to preclude any possibility of the presence or addition of one or more other features, numbers, steps, elements, or combinations thereof. In addition, the term "connection" used herein is used to include both indirect and direct connections of a plurality of elements.

Further, in the following description of the present invention, detailed descriptions of known functions or configurations incorporated herein will be omitted when they may make the gist of the present invention unnecessarily unclear.

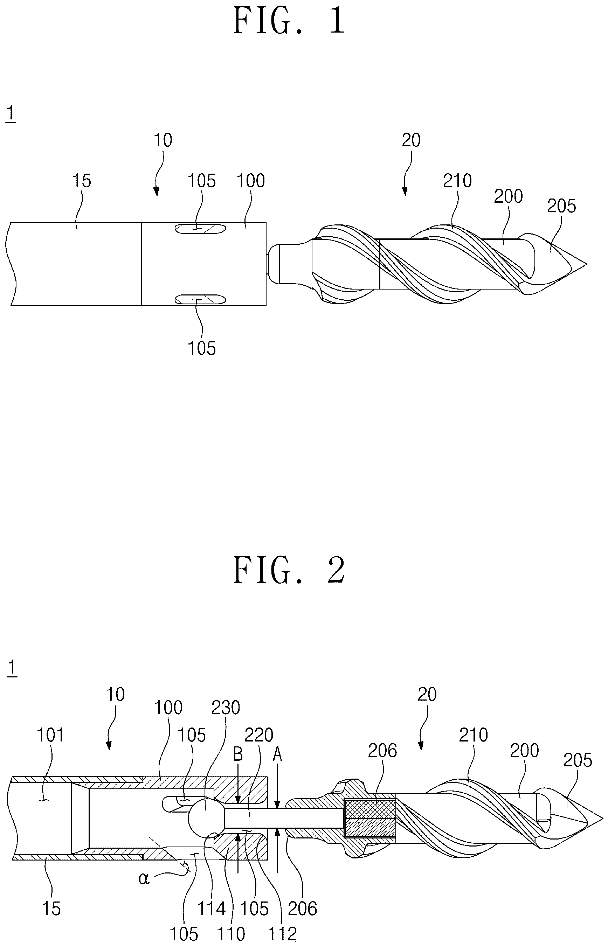

FIG. 1 is a view showing a magnetic robot according to one embodiment of the present invention, and FIG. 2 is a view showing a section of one portion of the magnetic robot according to one embodiment of the present invention.

Referring to FIGS. 1 and 2, according to one embodiment of the present invention, a magnetic robot 1 may include a body 10 and a work member 20.

According to one embodiment of the present invention, the magnetic robot 1 may be inserted into a blood vessel of a living body to remove foreign substances such as a thrombus. Hereinafter, regarding a direction in which the body 10 and the work member 20 are connected to each other, a direction in which the work member 20 is positioned will be defined as a front side, and a direction in which the body 10 is positioned will be defined as a rear side.

The body 10 may include a housing part 100 and a connection part 110.

The housing part 100 may have a preset length in a forward-rearward direction so as to form an inner space 101. An outer circumference of the housing part 100, which is perpendicular to a longitudinal direction of the housing part 100, may have a shape such as a circular shape or a polygonal shape. The inner space 101 of the housing part 100 may be opened rearward. When the magnetic robot 1 is used, a tube 15 may be connected to a posterior region of the housing part 100. At least one communication hole 105 may be formed at the outer circumference of the housing part 100. The communication hole 105 may be inclined forward at a preset angle $\alpha$ from the inner space to an outside. The communication hole 105 may have a preset length in a direction intersecting a circumferential direction of the housing part 100. For example, the communication hole 105 may have a preset length in the forward-rearward direction.

The connection part 110 may be positioned at a front end portion of the housing part 100 so as to protrude toward a longitudinal central axis of the housing part 100. Surfaces of the connection part 110, which face each other in a direction perpendicular to the longitudinal direction of the housing part 100, may be spaced apart from each other by a preset length, and a connection hole 115 may be formed at an inner center of the connection part 110. A section of the connection hole 115, which is perpendicular to a longitudinal direction of the connection hole 115, may have a circular shape. The connection hole 115 may have a preset length in the forward-rearward direction. A diameter of the connection hole 115 may be larger in a front end portion 112 of the connection part 110 than in a longitudinal central region of the connection part 110. For example, the front end portion 112 of the connection part 110 may have a structure that widens outward from a rear side to a front side. In addition, the front end portion 112 of the connection part 110 may have a structure that widens outward while being rounded with a preset curvature. A diameter of the connection hole 115 may be larger in a rear end portion 114 of the connection part 110 than in a longitudinal central region of the connection part 110. For example, the rear end portion 114 of the connection part 110 may have a structure that widens outward from a front side to a rear side. In addition, the rear end portion 114 of the connection part 110 may have a structure that widens outward while being rounded with a preset curvature.

The work member 20 may be connected to the body 10 so as to be positioned in an anterior region of the body 10. The work member 20 may have a preset length, and may be provided to be rotatable about a longitudinal center of the work member 20 so as to perform a function of removing a thrombus or the like attached to an inner side of a blood vessel. The work member 20 may include a body part 200, a fastening part 220, and a separation prevention part 230.

The body part 200 may have a preset length in the forward-rearward direction. An outer circumference of the body part 200, which is perpendicular to a longitudinal direction of the body part 200, may have a circular shape or the like. A blade 210 may be wound in a spiral shape around the outer circumference of the body part 200. A pointed portion 205 that is pointed forward may be formed at a front end of the body part 200. A rear end portion 206 of the body part 200 may be rounded. In addition, an outer circumference of the rear end portion 206 of the body part 200, which is perpendicular to a longitudinal direction of the rear end portion 206 of the body part 200, may have a circular shape, so that at least a portion of the rear end portion 206 of the body part 200 may inserted into the front end portion 112 of the connection part 110. At least one region of the body part 200 may be provided with a magnetic body 206. For example, the magnetic body 206 may be accommodated inside the body part 200. The magnetic body 206 may have N and S poles arranged in a direction orthogonal to the longitudinal direction of the body part 200. Accordingly, when a magnetic field is applied from the outside, a force may be generated in the magnetic body 206 so that the work member 20 may move inside the blood vessel while performing a drilling motion. In this case, a torque generated through the region in the magnetic body 206 may be expressed as Mathematical Formula 1.

[Mathematical Formula 1]

$$T_e = m \times B_e$$

(magnetic field: Be, Te: torque, m: magnetic moment of magnetic body)

In addition, in order to generate the drilling motion, the magnetic field may be applied in the following form.

[Mathematical Formula 2]

$$B_e(t) = B_0(cos(2\pi ft)\, U + sin(2\pi ft)\, N \times U)$$

($B_0$: intensity of magnetic field, f: rotation frequency, N: unit vector of rotation axis, U: unit vector perpendicular to rotation axis)

The fastening part 220 may extend rearward from a rear end of the body part 200 so as to be inserted into the connection hole 115. A section of the fastening part 220, which is perpendicular to a longitudinal direction of the fastening part 220, may have a circular shape. A diameter A of the fastening part 220 may be smaller than a diameter B of a central region of the connection hole 115 by a preset length. In this case, a difference between the diameter A of the fastening part 220 and the diameter B of the central region of the connection hole 115 may be clearly distinguished from an assembly tolerance that is generally considered upon designing mechanical elements for mutual assembly of the mechanical elements, and may refer to a value that is larger than the assembly tolerance. Accordingly, when a longitudinal central axis of the fastening part 220 and a longitudinal central axis of the connection hole 115 are aligned to overlap each other, an outer surface of the fastening part 220 may be spaced apart from the connection hole 115. A length of the fastening part 220 may be longer than a length of the central region of the connection hole 115. The length of the fastening part 220 may be longer than a length of the connection hole 115.

The separation prevention part 230 may be coupled to a rear end of the fastening part 220 so as to be positioned in the inner space of the housing part 100. At least a partial region of the separation prevention part 230 may be configured such that a section of the partial region, which is perpendicular to a longitudinal direction of the work member 20, may be larger than an area of the connection hole 115, thereby preventing the fastening part 220 from being separated through the connection hole 115. A front end portion of the separation prevention part 230 may widen outward while being rounded from a front side to a rear side. For example, the separation prevention part 230 may have a ball shape.

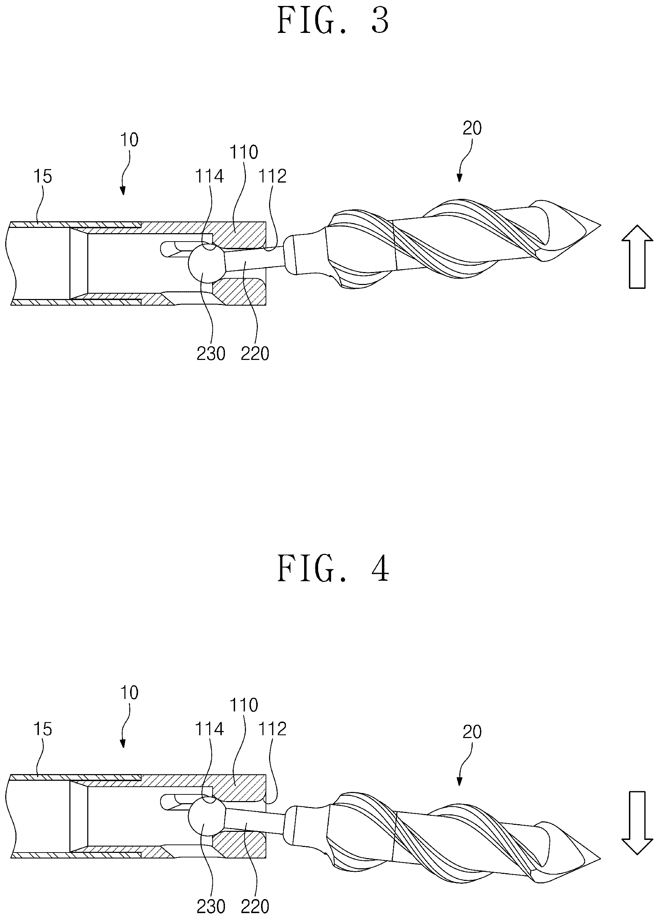

FIGS. 3 and 4 are views showing states where a direction in which a work member faces is adjusted.

Referring to FIGS. 3 and 4, a posture of the work member 20 may be adjusted such that the longitudinal direction of the work member 20 may be inclined with respect to a longitudinal direction of the body 10. For example, when an angle of the magnetic field applied to the work member 20 with respect to the longitudinal direction of the body 10 is adjusted, a direction in which the longitudinal direction of the work member 20 faces may be adjusted by a force generated by the magnetic field. In this case, when the longitudinal central axis of the fastening part 220 and the longitudinal central axis of the connection hole 115 are aligned to overlap each other, the outer surface of the fastening part 220 may be spaced apart from the connection hole 115, so that the posture of the work member 20 may be adjusted until a front end portion of the fastening part 220 and a rear end portion (or deviation prevention part) of the fastening part 220 make contact with the connection hole 115 in opposite directions. In addition, the front end portion 112 and the rear end portion 114 of the connection part 110 are may be rounded, so that even when the posture of the work member 20 is adjusted to allow the fastening part 220 or the separation prevention portion 230 to make contact with the connection part 110, damage caused by the contact may be prevented. Accordingly, according to the magnetic robot 1 of one embodiment of the present invention, steering ability may be improved by adjusting a direction of the work member 20 with respect to the body 10 during a process of moving forward after being inserted into the blood vessel.

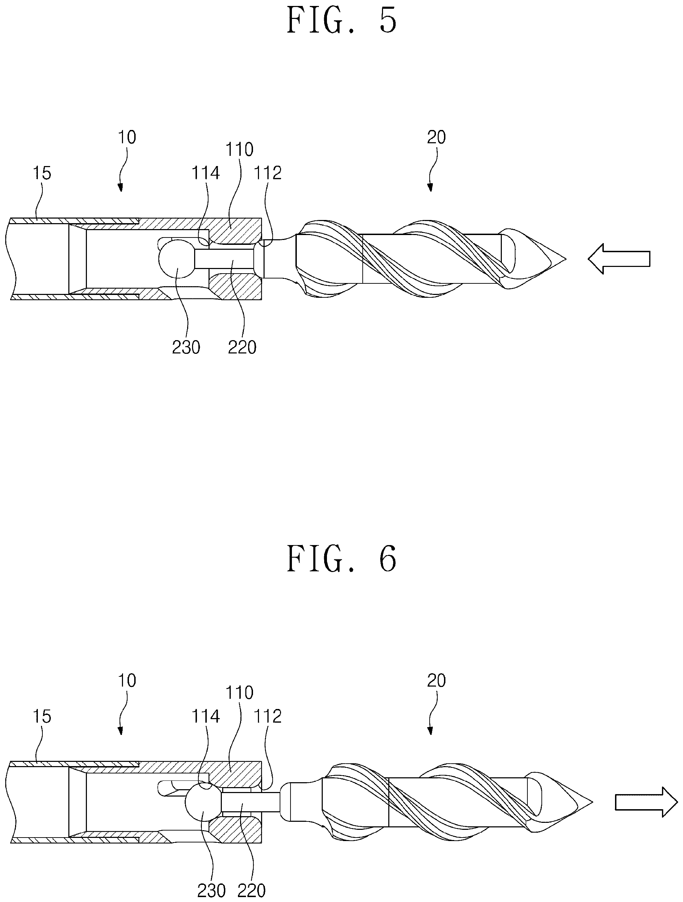

FIGS. 5 and 6 are views showing states where a position of the work member is adjusted in a forward-rearward direction with respect to a body.

Referring to FIGS. 5 and 6, the work member 20 may be provided to be movable in the forward-rearward direction with respect to the body 10. For example, when a forward-rearward position of the magnetic field applied to the work member 20 with respect to the body 10 is adjusted, the work member 20 may be moved in the forward-rearward direction with respect to the body 10 by the force applied to the work member 20 by the magnetic field. Accordingly, when the magnetic robot 1 is inserted into a blood vessel to perform a procedure, the work may be performed while moving the work member 20 in the forward-rearward direction with respect to the body 10. For example, the work member 20 may be operated in a scheme of moving in the forward-rearward direction with respect to the body 10, or in a scheme of moving in the forward-rearward direction with respect to the body 10 and rotating about the longitudinal direction of the body 10. Accordingly, a force applied to the thrombus by the work member 20 may be increased so that the thrombus may be fragmented more effectively. In addition, a work for manipulating a position of the body 10 to precisely set a forward-rearward position of the work member 20 may be omitted in the procedure using the magnetic robot 1 so that work efficiency may be increased, and a total movement distance of the body 10 and the tube 15 connected thereto in the forward-rearward direction may be reduced so that a load applied to the blood vessel may be reduced during a process of moving the body 10 and the tube 15. In addition, a position of the work member 20 with respect to the body 10 may be aligned in a process of moving the work member 20 rearward to insert the rear end of the body part 200 into the front end portion 112 of the connection part 110, so that the work member 20 may be moved in the forward-rearward direction while the position of the work member 20 with respect to the longitudinal direction of the body 10 is adjusted.

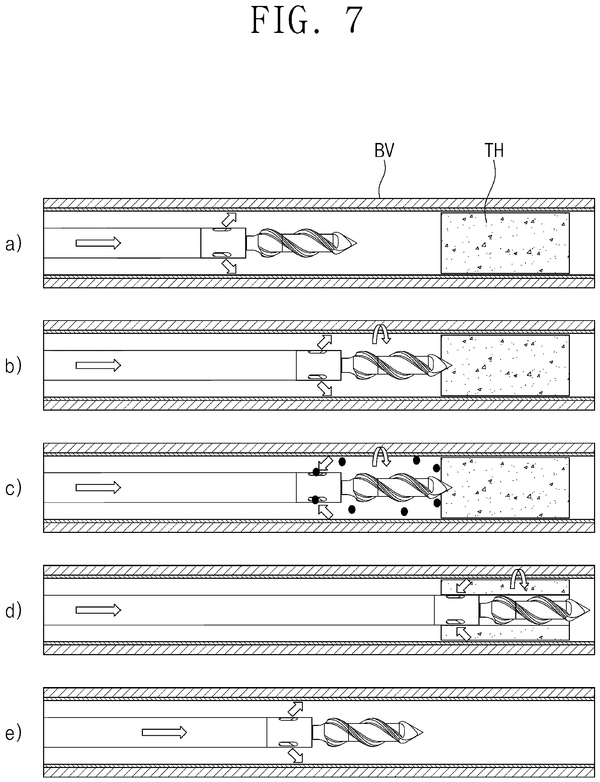

FIG. 7 is a view showing a method for inserting a magnetic robot into a blood vessel to perform a work.

Referring to FIG. 7, first, the magnetic robot 1 may be moved to a region in which a thrombus TH is positioned in a blood vessel BV for a medical procedure (a). The magnetic robot 1 may be steered by a force pushing the tube 15 into the blood vessel TH, which is a force applied to the work member 20, to move through an inside of the blood vessel TH. After the magnetic robot 1 arrives at a position adjacent to the thrombus TH, or during a process of moving to a work position, a medicine may be supplied into the body 10, so that the medicine may be discharged through the communication hole 105. The medicine may be a contrast medium or a medicine used for removing a thrombus.

Thereafter, the magnetic robot 1 may move to a region in which the thrombus TH is positioned, and the work member 20 may rotate through a magnetic field, or the work member 20 may rotate while moving in the forward-rearward direction with respect to the body 10, so that the thrombus may be fragmented through a drilling work (b, c, and d). The drilling work may be performed while the body 10 is fixed or while the body 10 is moved forward. In addition, a medicine may be supplied into the body 10 during the drilling work, so that the medicine may be discharged through the communication hole 105. Accordingly, the medicine may be a medicine used for removing a thrombus. In addition, during the drilling work through the rotation of the work member 20, suction and external discharge of debris generated inside the blood vessel during the thrombus fragmenting process may be performed by suction through the communication hole 105. During the drilling work, alternation between medicine discharge and debris suction through the communication hole 105 may be performed at least once. The magnetic robot 1 may perform the drilling work by moving forward to a point where a removal target thrombus is completely fragmented by the work member 20.

Thereafter, when the fragmentation of the thrombus through the rotation of the work member 20 is completed, the magnetic robot 1 may move rearward so as to be discharged out of the blood vessel through a point where the magnetic robot 1 was first inserted into the blood vessel (e). In addition, the magnetic robot 1 may perform the medicine discharge or the debris suction through the communication hole 105 before or during the process of moving rearward after the fragmentation of the thrombus through the rotation of the work member 20.

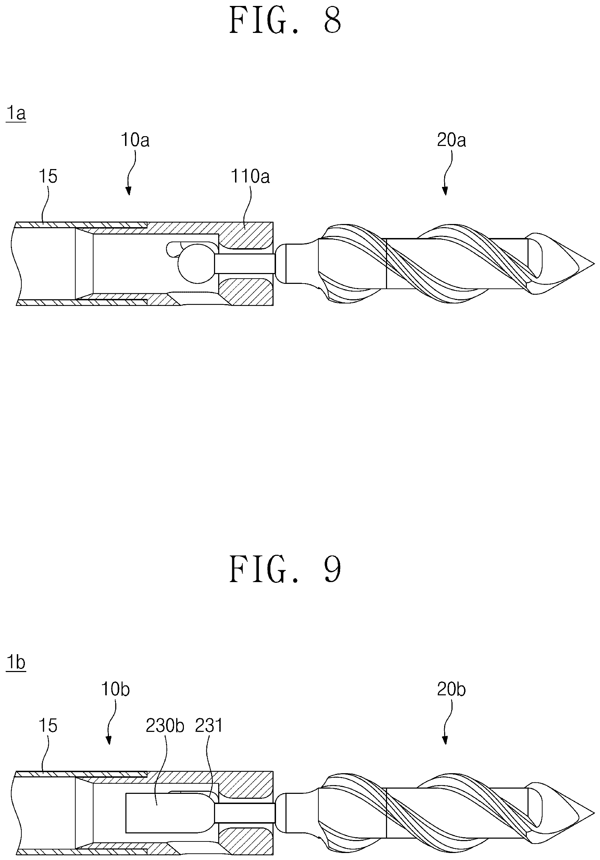

FIG. 8 is a view showing a magnetic robot according to a second embodiment of the present invention.

Referring to FIG. 8, a magnetic robot 1a may include a body 10a and a work member 20a.

The body 10a may include a housing part 100a and a connection part 110a.

The connection part 110a may be positioned at a front end portion of the housing part 100a so as to protrude toward a longitudinal central axis of the housing part 100a. Surfaces of the connection part 110a, which face each other in a direction perpendicular to a longitudinal direction of the housing part 100a, may be spaced apart from each other by a long set length, and a connection hole may be formed at an inner center of the connection part 110a. The connection hole may have the same area from a front end to a rear end of the connection hole.

Structures of the body 10a and the work member 20a may be identical or similar to the structures in the magnetic robot 1 of FIG. 1 except for a structure of the connection part 110a, so that redundant descriptions thereof will be omitted.

FIG. 9 is a view showing a magnetic robot according to a third embodiment of the present invention.

Referring to FIG. 9, a magnetic robot 1b may include a body 10b and a work member 20b.

At least a partial region of a separation prevention part 230b provided at a rear end portion of the work member 20b may be configured such that a section of the partial region, which is perpendicular to a longitudinal direction of the work member 20b, may be larger than an area of the connection hole, thereby preventing separation through the connection hole. The separation prevention part 230b may have a cylindrical column shape or a polygonal column shape. A front end portion 231 of the separation prevention part 230b may widen outward while being rounded from a front side to a rear side.

Structures of the body 10b and the work member 20b may be identical or similar to the structures in the magnetic robot 1 of FIG. 1 except for a structure of the separation prevention part 230b, so that redundant descriptions thereof will be omitted.

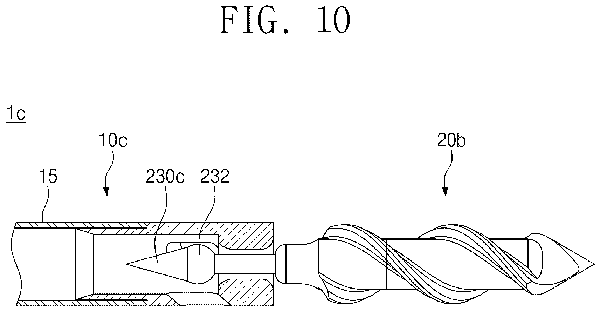

FIG. 10 is a view showing a magnetic robot according to a third embodiment of the present invention.

Referring to FIG. 10, a magnetic robot 1c may include a body 10c and a work member 20c.

At least a partial region of a separation prevention part 230c provided at a rear end portion of the work member 20c may be configured such that a section of the partial region, which is perpendicular to a longitudinal direction of the work member 20c, may be larger than an area of the connection hole, thereby preventing separation through the connection hole. The separation prevention part 230c may have a conical shape that is pointed rearward. A front end portion 232 of the separation prevention part 230c may widen outward while being rounded from a front side to a rear side.

Structures of the body 10c and the work member 20c may be identical or similar to the structures in the magnetic robot 1 of FIG. 1 except for a structure of the separation prevention part 230c, so that redundant descriptions thereof will be omitted.

Although the exemplary embodiments of the present invention have been described in detail above, the scope of the present invention is not limited to a specific embodiment, and shall be interpreted by the appended claims. In addition, it is to be understood by a person having ordinary skill in the art that various changes and modifications can be made without departing from the scope of the present invention.

[Industrial Applicability]

A magnetic robot according to an embodiment of the present invention may be used to treat tubular tissues inside a body, including blood vessels.

**Claims**

1. A magnetic robot comprising:

   a body; and
   a work member connected to the body so as to be positioned in an anterior region of the body, and provided to be rotatable about a longitudinal center of the work member,
   wherein the body includes:

      a housing part having a preset length in a forward-rearward direction so as to form an inner space; and
      a connection part positioned at a front end portion of the housing part so as to protrude toward a longitudinal central axis of the housing part, and having a connection hole formed at an inner center of the connection part, and

   the work member includes:

      a body part having a preset length in the forward-rearward direction, and having at least one region provided with a magnetic body; and
      a fastening part extending rearward from a rear end of the body part so as to be inserted into the connection hole.

2. The magnetic robot of claim 1, wherein the work member further includes:
   a separation prevention part coupled to a rear end of the fastening part so as to be positioned in the inner space of the housing part, and having at least a partial region in which a section of the partial region, which is perpendicular to a longitudinal direction of the work member, is larger than an area of the connection hole.

3. The magnetic robot of claim 1, wherein a section of the connection hole, which is perpendicular to a longitudinal direction of the connection hole, has a circular shape,

   a section of the fastening part, which is perpendicular to a longitudinal direction of the fastening part, has a circular shape, and
   a diameter of the fastening part is smaller than a diameter of a central region of the connection hole by a preset length.

4. The magnetic robot of claim 3, wherein a diameter of the connection hole is larger in a front end portion of the connection part than in a longitudinal central region of the connection part.

5. The magnetic robot of claim 4, wherein the front end portion of the connection part has a structure that widens outward from a rear side to a front side.

6. The magnetic robot of claim 3, wherein a diameter of the connection hole is larger in a rear end portion of the connection part than in a longitudinal central region of the connection part.

7. The magnetic robot of claim 6, wherein the rear end portion of the connection part has a structure that widens outward from a front side to a rear side.

8. The magnetic robot of claim 1, wherein at least one communication hole is formed at an outer circumference of the housing part.

9. The magnetic robot of claim 8, wherein the communication hole is inclined forward at a preset angle from the inner space to an outside.

# FIG. 1

# FIG. 2

# FIG. 3

20

10

15    114  110  112

230  220

# FIG. 4

10

110

15    114      112

20

230  220

# FIG. 5

# FIG. 6

# FIG. 7

# FIG. 8

1a

15    10a    110a    20a

# FIG. 9

1b

15    10b    230b    231    20b

# FIG. 10

## INTERNATIONAL SEARCH REPORT

| International application No. |
|---|
| **PCT/KR2023/002887** |

---

**A.     CLASSIFICATION OF SUBJECT MATTER**

**A61B 34/30**(2016.01)i; **A61B 17/22**(2006.01)i; **B25J 11/00**(2006.01)i; **B25J 15/00**(2006.01)i; **B25J 15/02**(2006.01)i

According to International Patent Classification (IPC) or to both national classification and IPC

**B.     FIELDS SEARCHED**

Minimum documentation searched (classification system followed by classification symbols)

A61B 34/30(2016.01); A61B 17/00(2006.01); A61B 34/00(2016.01); A61B 34/20(2016.01); A61F 2/958(2013.01); A61M 25/00(2006.01); A61M 25/09(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 마그네틱 로봇(magnetic robot), 회전부재(rotary member), 체결부재(fastening member), 볼 소켓(ball socket), 연통홀(communication holes)

**C.     DOCUMENTS CONSIDERED TO BE RELEVANT**

| Category* | Citation of document, with indication, where appropriate, of the relevant passages | Relevant to claim No. |
|---|---|---|
| X | KR 10-2016-0100853 A (DAEGU GYEONGBUK INSTITUTE OF SCIENCE AND TECHNOLOGY) 24 August 2016 (2016-08-24)<br>     See paragraphs [0021]-[0024] and [0032]; and figures 1 and 4. | 1-3,8 |
| A | | 4-7,9 |
| A | KR 10-2019-0135330 A (INDUSTRY FOUNDATION OF CHONNAM NATIONAL UNIVERSITY) 06 December 2019 (2019-12-06)<br>     See entire document. | 1-9 |
| A | US 2014-0100646 A1 (ACCESSCLOSURE, INC.) 10 April 2014 (2014-04-10)<br>     See entire document. | 1-9 |
| A | KR 10-2021-0098200 A (IUCF-HYU (INDUSTRY-UNIVERSITY COOPERATION FOUNDATION HANYANG UNIVERSITY)) 10 August 2021 (2021-08-10)<br>     See entire document. | 1-9 |

| ✓ Further documents are listed in the continuation of Box C. | ✓ See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **07 June 2023** | **07 June 2023** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

International application No.

**PCT/KR2023/002887**

**C.     DOCUMENTS CONSIDERED TO BE RELEVANT**

| Category* | Citation of document, with indication, where appropriate, of the relevant passages | Relevant to claim No. |
|---|---|---|
| A | KR 10-2021-0012852 A (IUCF-HYU (INDUSTRY-UNIVERSITY COOPERATION FOUNDATION HANYANG UNIVERSITY)) 03 February 2021 (2021-02-03)<br>See entire document. | 1-9 |

Form PCT/ISA/210 (second sheet) (July 2022)

## INTERNATIONAL SEARCH REPORT
### Information on patent family members

International application No.

**PCT/KR2023/002887**

| Patent document cited in search report | | | Publication date (day/month/year) | Patent family member(s) | | | Publication date (day/month/year) |
|---|---|---|---|---|---|---|---|
| KR | 10-2016-0100853 | A | 24 August 2016 | JP | 2016-150257 | A | 22 August 2016 |
| | | | | JP | 6196340 | B2 | 13 September 2017 |
| | | | | KR | 10-1659367 | B1 | 23 September 2016 |
| | | | | KR | 10-1845941 | B1 | 05 April 2018 |
| | | | | KR | 10-2016-0101442 | A | 25 August 2016 |
| | | | | US | 2016-0235491 | A1 | 18 August 2016 |
| | | | | US | 9968411 | B2 | 15 May 2018 |
| KR | 10-2019-0135330 | A | 06 December 2019 | KR | 10-2274982 | B1 | 09 July 2021 |
| | | | | US | 2021-0220068 | A1 | 22 July 2021 |
| | | | | WO | 2019-231088 | A1 | 05 December 2019 |
| US | 2014-0100646 | A1 | 10 April 2014 | CA | 2885769 | A1 | 17 April 2014 |
| | | | | CN | 104918587 | A | 16 September 2015 |
| | | | | CN | 104918587 | B | 04 May 2018 |
| | | | | EP | 2906282 | A1 | 19 August 2015 |
| | | | | JP | 2015-531283 | A | 02 November 2015 |
| | | | | KR | 10-2015-0067169 | A | 17 June 2015 |
| | | | | WO | 2014-058797 | A1 | 17 April 2014 |
| | | | | ZA | 201503094 | B | 26 October 2016 |
| KR | 10-2021-0098200 | A | 10 August 2021 | CN | 115038394 | A | 09 September 2022 |
| | | | | EP | 4085850 | A1 | 09 November 2022 |
| | | | | US | 2023-0050583 | A1 | 16 February 2023 |
| | | | | WO | 2021-153956 | A1 | 05 August 2021 |
| KR | 10-2021-0012852 | A | 03 February 2021 | CN | 114173697 | A | 11 March 2022 |
| | | | | EP | 4005518 | A1 | 01 June 2022 |
| | | | | KR | 10-2348773 | B1 | 07 January 2022 |
| | | | | US | 2022-0280182 | A1 | 08 September 2022 |
| | | | | WO | 2021-020751 | A1 | 04 February 2021 |

Form PCT/ISA/210 (patent family annex) (July 2022)